# EUROPEAN PATENT SPECIFICATION

(11) **EP 2 741 777 B1**
(45) Date of publication and mention of the grant of the patent: **18.01.2017**
(21) Application number: 12745493.2
(22) Date of filing: 10.08.2012
(51) Int. Cl.: A61P 11/00, A61K 47/00

(54) **METHODS AND PHARMACEUTICAL COMPOSITIONS FOR TREATMENT OF PULMONARY HYPERTENSION**
VERFAHREN UND PHARMAZEUTISCHE ZUSAMMENSETZUNGEN ZUR BEHANDLUNG VON PULMONALER HYPERTONIE
PROCÉDÉS ET COMPOSITIONS PHARMACEUTIQUES POUR LE TRAITEMENT DE L'HYPERTENSION PULMONAIRE

(30) Priority: 12.08.2011 EP 11306042
(43) Date of publication of application: 18.06.2014
(73) Proprietor: INSERM - Institut National de la Santé et de la Recherche Médicale, 75013 Paris (FR); Université Paris-Est Créteil Val de Marne, 94010 Creteil Cedex (FR); Assistance Publique Hôpitaux de Paris, 75004 Paris (FR)
(72) Inventor: ADNOT, Serge, F-94010 Creteil Cedex (FR); AMSELLEM, Valérie, F-94000 Créteil (FR)
(74) Representative: Hirsch, Denise Marie
(86) International application number: PCT/EP2012/065672
(87) International publication number: WO 2013/024022

(56) References cited:
- WO-A1-2008/070117
- WRIGHT J L ET AL: "Animal models of cigarette smoke-induced chronic obstructive pulmonary disease", EXPERT REVIEW OF RESPIRATORY MEDICINE, EXPERT REVIEWS, GB, vol. 4, no. 6, 1 January 2010 (2010-01-01) , pages 723-734, XP009152972, ISSN: 1747-6348
- KARSHOVSKA E ET AL: "Mechanisms of arterial remodeling and neointima formation: an updated view on the chemokine system", DRUG DISCOVERY TODAY: DISEASE MECHANISMS, ELSEVIER, vol. 5, no. 3-4, 1 January 2008 (2008-01-01), pages E293-E298, XP009152944, ISSN: 1740-6765
- LIU TAO ET AL: "Recent Advances in the Development of Small-molecule CCR5 Inhibitors for HIV", MINI REVIEWS IN MEDICINAL CHEMISTRY, BENTHAM SCIENCE PUBLISHERS, HILVERSUM, NL, vol. 10, no. 13, 1 November 2010 (2010-11-01), pages 1277-1292, XP009152954, ISSN: 1389-5575
- AMSELLEM ET AL: "CCR5 as a target in Pulmonary Arterial Hypertnsion.", CIRCULATION, vol. 130, 2014, - 2014, pages 880-891,

## Description

### FIELD OF THE INVENTION:

The present invention relates to uses, methods and compositions for the treatment of pulmonary hypertension.

### BACKGROUND OF THE INVENTION:

Pulmonary hypertension (PH) is a fatal disease caused by small pulmonary artery obstruction from vascular proliferation and remodeling. PH is characterized by elevated pulmonary arterial pressure and increased pulmonary vascular resistance, frequently leading to right-sided heart failure and death (Fukumoto and Shimokawa, 2011).

Pulmonary arterial hypertension (PAH), whether idiopathic or associated with underlying diseases, is an unexplained condition which results from structural remodeling of the pulmonary vessels. Persistent vasoconstriction, proliferation of vascular smooth muscle, extracellular matrix accumulation, and lung infiltration by inflammatory cells are important components of this remodelling (Raiesdana and Loscalzo, 2006; Simonneau et al., 2004).

Severe PAH develops in a substantial number of patients with HIV infection and is considered as a classical complication of HIV infection. HIV associated PAH has gained importance following the improved survival of HIV-infected patients, it is associated with a particularly poor prognosis and decreased survival compared with HIV-infected patients without PAH (Raiesdana and Loscalzo, 2006; Simonneau et al., 2004).

There is no disclosure in the art of the use of CCR5 antagonist or inhibitor of CCR5 expression in methods for inhibiting smooth muscle cell proliferation, nor the use of CCR5 antagonist or inhibitor of CCR5 expression in methods for treatment of pulmonary hypertension and pulmonary arterial hypertension.

However, there is a need to develop new drugs that will be suitable for treatment of pulmonary hypertension.

### SUMMARY OF THE INVENTION:

The first object of the invention relates to a compound which is a CCR5 antagonist and/or an inhibitor of CCR5 expression for use in the prevention or treatment of pulmonary hypertension in a subject in need thereof.

Another object of the invention relates to a compound which is a CCR5 antagonist and/or an inhibitor of CCR5 expression for use in a method for inhibiting smooth muscle cell proliferation in a subject afflicted with pulmonary hypertension.

Another object of the invention relates to a compound which is a CCR5 inhibitor and/or an inhibitor of CCR5 expression for use in the prevention or treatment of vascular remodeling in a subject in need thereof.

In a particular embodiment said subject is afflicted with pulmonary arterial hypertension.

In a particular embodiment said subject is afflicted with HIV infection.

Another object of the invention relates to a pharmaceutical composition comprising a compound according to the invention and a pharmaceutical acceptable carrier.

Another object of the invention relates to a method of identifying a subject having or at risk of having or developing a pulmonary hypertension.

Another object of the invention relates to a method according to the invention for monitoring the efficacy of a treatment for a pulmonary hypertension.

### DETAILED DESCRIPTION OF THE INVENTION:

The role of CCR5 in pulmonary hypertension was investigated by the inventors using CCR5 knock out mice, CCR5 knock-in mice, experimental PAH induction by exposure to chronic hypoxia and human pulmonary-artery smooth muscle cells collected from lung tissue samples of patients undergoing lung resection surgery. The inventors surprisingly found that CCR5 knock out mice are protected against development of PAH induced by exposure to chronic hypoxia. The inventors also demonstrate that CCR5 is involved in muscularization of pulmonary arteries during exposure to chronic hypoxia, then CCR5 deficiency attenuate the development of PAH in CCR5 -/- mice with an associated reduction in lung inflammatory cell infiltrates and cytokines expression. The inventors also demonstrated that treatment with the CCR5 antagonist maraviroc protects against hypoxic pulmonary hypertension in CCR5 knock-in mice but not in wild-type mice. The inventors also demonstrated that proliferation of human pulmonary artery smooth muscle cells stimulated by the CCR5 ligand CCL5 is significantly reduced by pharmacological inhibition of CCR5 with maraviroc.

### Definitions

The term "CCR5" has its general meaning in the art and refers to CC motif receptor 5 or CC-chemokine receptor 5 (CCR5).

As used herein, the term "subject" denotes a mammal. In a preferred embodiment of the invention, a subject according to the invention refers to any subject (preferably human) afflicted with pulmonary hypertension. In a particular embodiment, the subject is afflicted with pulmonary arterial hypertension. In a particular embodiment, the subject is afflicted with pulmonary arterial hypertension associated HIV infection.

In the context of the invention, the term "treating" or "treatment", as used herein, refers to a method that is aimed at reversing, alleviating, inhibiting, slowing down or stopping the progression, aggravation or deterioration of the symptoms of the pathology, at bringing about ameliorations of the symptoms of the pathology, and/or at curing the pathology. The terms "preventing" or "prevention", as used herein, refer to a method that is aimed at delaying or preventing the onset of the pathology.

By "CCR5 antagonist" is meant a natural or synthetic compound that has a biological effect opposite to that of a natural ligand of the receptor. An antagonist binds the receptor and blocks the action of the natural ligand by competing with the ligand for receptor binding. An antagonist is defined by its ability to block the actions of a natural ligand. The term "CCR5 antagonist" refers to any CCR5 antagonist that is currently known in the art or that will be identified in the future, and includes any chemical entity that, upon administration to a subject, results in inhibition of a biological activity associated with activation of the CCR5 in the subject, including any of the downstream biological effects otherwise resulting from the binding to CCR5 of its natural ligands. Such CCR5 antagonist includes any agent that can block CCR5 activation or any of the downstream biological effects of CCR5 activation. Such an antagonist can act by binding directly to the intracellular or extracellular domain of the receptor and inhibiting its activity and its intracellular G-protein signaling. Alternatively, such an antagonist can act by occupying the ligand binding site or a portion thereof of the CCR5 receptor, thereby making the receptor inaccessible to its natural ligand so that its normal biological activity is prevented or reduced. Alternatively, such an inhibitor can act by modulating the dimerization of CCR5 polypeptides, or interaction of CCR5 polypeptide with other proteins. Therefore the term "CCR5 antagonist" refers to an antagonist of the CCR5 protein. In the context of the present invention, CCR5 antagonists of the invention are preferably selective for CCR5 as compared with the related chemokine receptors. By "selective" it is meant that the affinity of the CCR5 antagonist is at least 10-fold, preferably 25-fold, more preferably 100-fold, still preferably 500-fold higher than the affinity for the related chemokine receptors.

### Therapeutic methods and uses

The present invention relates to a compound which is a CCR5 antagonist and/or an inhibitor of CCR5 expression for use in the prevention or treatment of pulmonary hypertension in a subject in need thereof.

The method of the invention may be performed for any type of pulmonary hypertension such as revised in the World Health Organisation Classification of pulmonary hypertension and selected from the group consisting of Pulmonary arterial hypertension that develops as sporadic disease (idiopathic), as an inherited disorder (familial), or in association with certain conditions (collagen vascular diseases, congenital systemic-to-pulmonary shunts (large, small, repaired, or nonrepaired), portal hypertension, human immunodeficiency virus (HIV) infection, ingestion of drugs or dietary products and toxins (anorexigens, rapeseed oil, L-tryptophan, methamphetamine, and cocaine), or in association with other conditions (thyroid disorders, glycogen storage disease, Gaucher disease, hereditary hemorrhagic telangiectasia, hemoglobinopathies, myeloproliferative disorders, and splenectomy)), or associated with significant venous or capillary involvement (pulmonary veno-occlusive disease and pulmonary capillary hemangiomatosis) and persistent pulmonary hypertension of the newborn; Pulmonary venous hypertension (left-sided atrial or ventricular heart disease, left-sided valvular heart disease); Pulmonary hypertension associated with hypoxemia (chronic obstructive pulmonary disease, Interstitial lung disease; Sleep-disordered breathing, alveolar hypoventilation disorders, Long-term exposure to high altitudes, developmental abnormalities); Pulmonary hypertension due to chronic thrombotic or embolic disease (thromboembolic obstruction of proximal pulmonary arteries, thromboembolic obstruction of distal pulmonary arteries, Pulmonary embolism (tumor, parasites, foreign material)); Miscellaneous (sarcoidosis, pulmonary Langerhans'-cell histiocytosis, lymphangiomatosis, compression of pulmonary vessels (adenopathy, tumor, fibrosing mediastinitis) (Raiesdana and Loscalzo, 2006; Simonneau et al., 2004).

In one embodiment, the CCR5 antagonist is a low molecular weight antagonist, e. g. a small organic molecule.

The term "small organic molecule" refers to a molecule of a size comparable to those organic molecules generally used in pharmaceuticals. The term excludes biological macromolecules (e. g., proteins, nucleic acids, etc.). Preferred small organic molecules range in size up to about 5000 Da, more preferably up to 2000 Da, and most preferably up to about 1000 Da.

Said CCR5 antagonists are well-known as illustrated by Singh and Chauthe, (2011), Ness et al., (2006) and Yang and Rotstein, (2009).

In one embodiment, the CCR5 antagonist is selected in the group consisting of aryl oxime-piperazine derivatives (see, e.g., U.S. Pat. No. 6,689,783), piperazine derivatives (see, e.g., U.S. Pat. No. 6,689,765), piperidine derivatives (see, e.g., U.S. Pat. No. 6,602,885), pyrrole derivatives (see, e.g., U.S. Pat. No. 6,562,859), pyrrolidine derivatives (see, e.g., U.S. Pat. No. 6,531,484), anilide derivatives (see, e.g., U.S. Pat. No. 6,235,771), bis-acridines (see, e.g., U.S. Pat. No. 6,242, 459), and azabicycloalkanes derivatives (International Publication No. WO 00/38680).

Other examples of CCR5 antagonist include but are not limited to the compounds disclosed in WO99/17773, WO99/32100, WO00/06085, WO00/06146, WO00/10965, WO00/06153, WO00/21916, WO00/37455, EP1013276, WO00/38680, WO00/39125, WO00/40239, WO00/42045, WO00/53175, WO00/42852, WO00/66551, WO00/66558, WO00/66559, WO00/66141, WO00/68203, JP2000309598, WO00/51607, WO00/51608, WO00/51609, WO00/51610, WO00/56729, WO00/59497, WO00/59498, WO00/59502, WO00/59503, WO00/76933, WO98/25605, WO99/04794, WO99/38514, or the compounds disclosed in Forbes et al., 2000.

In one embodiment of the invention, the CCR5 antagonists is selected from maraviroc (4,4-difluoro-*N*-{(1*S*)-3-[3-(3-isopropyl-5-methyl-4*H*-1,2,4-triazol-4-yl)-8-azabicyclo[3.2.1]oct-8-yl]-1-phenylpropyl}cyclohexanecarboxamide) and derivatives such as those described in (WO 00/38680; US6,586,430; US6,667,313; US7,368,460; US7,576,097; US6,667,314), NCB- 9471 , PRO-140, TAK-779 (WO 99/32468), ZM-688523, 4-chloro-6-fluoro sulphonamide, UK-427857, TAK-220 (WO 01/25200), TAK-652 which is disclosed in WO03014105 and has the chemical name 8-[4-(2-butoxyethoxy)phenyl]-1-isobutyl-N-[4-[[(1-propyl-1 H-imadazol-5- yl)methyl]sulphinyl]phenyl]-1,2,3,4-tetrahydro-1-benzacocine-5-carboxamide, SC-351125, ancriviroc (formerly known as SCH-C), SCH-351125 (SCH-C), SCH-417690 (SCH-D), vicroviroc which has the chemical name (4,6-dimethylprymidine-5-yl){4-[(3S)-4-{(1R)-2-methoxy-1-[4-(trifluoromethyl)phenyl]ethyl}-3-methylpiperazin-1-yl]-4-methylpiperidin-1 - yl}methanone, PRO-140, apliviroc (formerly known as GW-873140, Ono-4128, AK-602), AMD-887, INC- B9471 , CMPD-167 which has the chemical name N-methyl-N-((1R,3S,4S)-3-[4-(3-benzyl-1-ethyl-1H-pyrazol-5-yl)piperidin-1-ylmethyl]-4-[3-fluorophenyl]cyclopent-1-yl]-D-valine), methyl1-endo-{8-[(3S)-3-(acetylamino)-3-(3-fluorophenyl)propyl]-8-azabicyclo[3.2.1]oct-3-yl}-2-methyl-4,5,6,7-tetrahydro-1 H- imidazo[4,5-c]pyridine-5-carboxylate, methyl 3-endo-{8-[(3S)-3-(acetamido)-3-(3-fluorophenyl)propyl]-8- azabicyclo[3.2.1]oct-3-yl}-2-methyl-4,5,6,7-tetrahydro-3H-imidazo[4,5-c]pyridine-5-carboxylate, ethyl 1- endo-{8-[(3S)-3-(acetylamino)-3-(3-fluorophenyl)propyl]-8-azabicyclo[3.2.1]oct-3-yl}-2-methyl-4,5,6,7- tetrahydro-1H-imidazo[4,5-c]pyridine-5-carboxylate and N-{(1S)-3-[3-endo-(5-Isobutyryl-2-methyl-4,5,6,7-tetrahydro-1H-imidazo[4,5-c]pyridin-1-yl)-8-azabicyclo[3.2.1]oct-8-yl]-1-(3-fluorophenyl)propyl}acetamide) and pharmaceutically acceptable salts, solvates or derivatives of the above (WO 03/084954; WO 05/033107; WO08/099278).

In another embodiment, the CCR5 antagonist may consist in a modified chemokines that can partially or completely block CCR5 activation. Said modified chemokines CCR5 antagonists are well-known as illustrated by Ness et al., (2006), U.S. Pat. No. 6,555,105 and U.S. Pat. No. 6,942,852.

Non-limiting examples of modified chemokines CCR5 antagonists include methiolynated RANTES (Met-RANTES), aminooxypentane-RANTES (AOP-RANTES), N^{α}-(*n*-nonanoyl)-*des*-Ser¹-RANTES (NNY-RANTES) and N^{α}-(*n*-nonanol)-*des*-Ser¹[L-thioproline², L-α-cyclohexyl-glycine³]-RANTES (PSC-RANTES) (Ness et al., 2006).

In another embodiment, the CCR5 antagonist may consist in an antibody or antibody fragment that can partially or completely block CCR5 activation.

Non-limiting examples of antibody-based CCR5 antagonists include CCR5mAb004, PRO 140 (Biswas et al., 2007), HGS004 and HGS101 (Latinovic et al., 2011), 2D7, PA9, PA14, MC-1, MC-4, MC-6, RoAb12, RoAb14, RoAb18 (Lopalco, 2011) and those described in Briihl et al., 2001, Schanzer et al., 2011, WO 2008/037419, WO2000/53633, WO 2006/103100, US 2004/0043033, US 6,610,834, US 2003/0228306, US 2003/0195348, US 2003/0166870, US 2003/0166024, US 2003/0165988, US 2003/0152913, US 2003/0100058, US 2003/0099645, US 2003/0049251, US 2003/0044411, US 2003/0003440, US 6,528,625, US 2002/0147147, US 2002/0146415, US 2002/0106374, US 2002/0061834, US 2002/0048786, US 2001/0000241, EP 1322 332, EP 1 263 791, EP 1 207 202, EP 1 161 456, EP 1 144 006, WO 2003/072766, WO 2003/066830, WO 2003/033666, WO 2002/083172, WO 02/22077, WO 01/58916, WO 01/58915, WO 01/43779 and WO 01/42308.

In one embodiment of the antibodies or portions thereof described herein, the antibody is a monoclonal antibody. In one embodiment of the antibodies or portions thereof described herein, the antibody is a polyclonal antibody. In one embodiment of the antibodies or portions thereof described herein, the antibody is a humanized antibody. In one embodiment of the antibodies or portions thereof described herein, the antibody is a chimeric antibody. In one embodiment of the antibodies or portions thereof described herein, the portion of the antibody comprises a light chain of the antibody. In one embodiment of the antibodies or portions thereof described herein, the portion of the antibody comprises a heavy chain of the antibody. In one embodiment of the antibodies or portions thereof described herein, the portion of the antibody comprises a Fab portion of the antibody. In one embodiment of the antibodies or portions thereof described herein, the portion of the antibody comprises a F(ab')2 portion of the antibody. In one embodiment of the antibodies or portions thereof described herein, the portion of the antibody comprises a Fc portion of the antibody. In one embodiment of the antibodies or portions thereof described herein, the portion of the antibody comprises a Fv portion of the antibody. In one embodiment of the antibodies or portions thereof described herein, the portion of the antibody comprises a variable domain of the antibody. In one embodiment of the antibodies or portions thereof described herein, the portion of the antibody comprises one or more CDR domains of the antibody.

As used herein, "antibody" includes both naturally occurring and non-naturally occurring antibodies. Specifically, "antibody" includes polyclonal and monoclonal antibodies, and monovalent and divalent fragments thereof. Furthermore, "antibody" includes chimeric antibodies, wholly synthetic antibodies, single chain antibodies, and fragments thereof. The antibody may be a human or nonhuman antibody. A nonhuman antibody may be humanized by recombinant methods to reduce its immunogenicity in man.

Antibodies are prepared according to conventional methodology. Monoclonal antibodies may be generated using the method of Kohler and Milstein (1975). To prepare monoclonal antibodies useful in the invention, a mouse or other appropriate host animal is immunized at suitable intervals (e.g., twice-weekly, weekly, twice-monthly or monthly) with antigenic forms of CCR5. The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes.

Briefly, the antigen may be provided as synthetic peptides corresponding to antigenic regions of interest in CCR5. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

Significantly, as is well-known in the art, only a small portion of an antibody molecule, the paratope, is involved in the binding of the antibody to its epitope (see, in general, Clark, W. R. (1986) The Experimental Foundations of Modern Immunology Wiley & Sons, Inc., New York; Roitt, I. (1991) Essential Immunology, 7th Ed., Blackwell Scientific Publications, Oxford). The Fc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDRS). The CDRs, and in particular the CDRS regions, and more particularly the heavy chain CDRS, are largely responsible for antibody specificity.

It is now well-established in the art that the non CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody.

This invention provides in certain embodiments compositions and methods that include humanized forms of antibodies. As used herein, "humanized" describes antibodies wherein some, most or all of the amino acids outside the CDR regions are replaced with corresponding amino acids derived from human immunoglobulin molecules. Methods of humanization include, but are not limited to, those described in U.S. Pat. Nos. 4,816,567, 5,225,539, 5,585,089, 5,693,761, 5,693,762 and 5,859,205, which are hereby incorporated by reference. The above U.S. Pat. Nos. 5,585,089 and 5,693,761, and WO 90/07861 also propose four possible criteria which may used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3A of the CDRs in a three dimensional model of the antibody and is predicted to be capable of interacting with the CDRs. The above methods are merely illustrative of some of the methods that one skilled in the art could employ to make humanized antibodies. One of ordinary skill in the art will be familiar with other methods for antibody humanization.

In one embodiment of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgG1, IgG2, IgG3, IgG4, IgA and IgM molecules. A "humanized" antibody retains a similar antigenic specificity as the original antibody. However, using certain methods of humanization, the affinity and/or specificity of binding of the antibody may be increased using methods of "directed evolution", as described by Wu et al., (1999), the contents of which are incorporated herein by reference.

Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein, the contents of which are incorporated herein by reference. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans.

In vitro methods also exist for producing human antibodies. These include phage display technology (U.S. Pat. Nos. 5,565,332 and 5,573,905) and in vitro stimulation of human B cells (U.S. Pat. Nos. 5,229,275 and 5,567,610). The contents of these patents are incorporated herein by reference.

Thus, as will be apparent to one of ordinary skill in the art, the present invention also provides for F(ab') 2 Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

The various antibody molecules and fragments may derive from any of the commonly known immunoglobulin classes, including but not limited to IgA, secretory IgA, IgE, IgG and IgM. IgG subclasses are also well known to those in the art and include but are not limited to human IgG1, IgG2, IgG3 and IgG4.

In another embodiment, the antibody according to the invention is a single domain antibody. The term "single domain antibody" (sdAb) or "VHH" refers to the single heavy chain variable domain of antibodies of the type that can be found in Camelid mammals which are naturally devoid of light chains. Such VHH are also called "nanobody®". According to the invention, sdAb can particularly be llama sdAb.

Then after raising antibodies directed against CCR5s as above described, the skilled man in the art can easily select those inhibiting CCR5.

In another embodiment the inhibitor of CCR5 is an aptamer. Aptamers are a class of molecule that represents an alternative to antibodies in term of molecular recognition. Aptamers are oligonucleotide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996). Then after raising aptamers directed against CCR5s as above described, the skilled man in the art can easily select those inhibiting CCR5.

Another object of the invention relates to a compound which is an inhibitor of CCR5 expression for use in the prevention or treatment of pulmonary hypertension in a subject in need thereof.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins (e.g., CCR5) modified by, for example, methylation, acetylation, phosphorylation, ubiquitination, SUMOylation, ADP-ribosylation, myristilation, and glycosylation.

An "inhibitor of expression" refers to a natural or synthetic compound that has a biological effect to inhibit the expression of a gene.

Inhibitors of CCR5 expression for use in the present invention may be based on antisense oligonucleotide constructs. Anti-sense oligonucleotides, including anti-sense RNA molecules and anti-sense DNA molecules, would act to directly block the translation of CCR5 mRNA by binding thereto and thus preventing protein translation or increasing mRNA degradation, thus decreasing the level of CCR5 proteins, and thus activity, in a cell. For example, antisense oligonucleotides of at least about 15 bases and complementary to unique regions of the mRNA transcript sequence encoding CCR5 can be synthesized, e.g., by conventional phosphodiester techniques and administered by e.g., intravenous injection or infusion. Methods for using antisense techniques for specifically alleviating gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of CCR5 expression for use in the present invention. CCR5 gene expression can be reduced by contacting the subject or cell with a small double stranded RNA (dsRNA), or a vector or construct causing the production of a small double stranded RNA, such that CCR5 expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of CCR5 expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of CCR5 mRNA sequences are thereby useful within the scope of the present invention. Specific ribozyme cleavage sites within any potential RNA target are initially identified by scanning the target molecule for ribozyme cleavage sites, which typically include the following sequences, GUA, GuU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of CCR5 expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing CCR5. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Another object of the invention relates to a compound which is a CCR5 antagonist and/or an inhibitor of CCR5 expression for use in a method for inhibiting smooth muscle cell proliferation in a subject afflicted with pulmonary hypertension.

Another object of the invention relates to a compound which is a CCR5 inhibitor and/or an inhibitor of CCR5 expression for use in the prevention or treatment of vascular remodeling in a subject afflicted with a pulmonary hypertension.

In a particular embodiment said subject is afflicted with pulmonary arterial hypertension.

In a particular embodiment said subject is afflicted with HIV infection.

In another embodiment, the present invention relates to a method of preventing or treating pulmonary hypertension in a subject in need thereof, comprising the step of administering to said subject a compound which is a CCR5 antagonist and/or an inhibitor of CCR5 expression.

In one embodiment said CCR5 antagonist is maraviroc.

### Pharmaceutical composition

Another object of the invention relates to a pharmaceutical composition comprising a CCR5 antagonist and/or an inhibitor of CCR5 expression and a pharmaceutically acceptable carrier for use in the prevention or treatment of pulmonary hypertension in a subject in need thereof.

Another object of the invention relates to a pharmaceutical composition comprising a CCR5 antagonist and/or an inhibitor of CCR5 expression and a pharmaceutically acceptable carrier for use in a method for inhibiting smooth muscle cell proliferation in a subject afflicted with pulmonary hypertension.

Another object of the invention relates to a pharmaceutical composition comprising a CCR5 antagonist and/or an inhibitor of CCR5 expression and a pharmaceutically acceptable carrier for use in the prevention or treatment of vascular remodeling in a subject afflicted with pulmonary hypertension.

Typically, CCR5 antagonist and/or an inhibitor of CCR5 expression may be combined with pharmaceutically acceptable excipients, and optionally sustained-release matrices, such as biodegradable polymers, to form therapeutic compositions.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The CCR5 antagonist and/or the inhibitor of CCR5 expression can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

Pharmaceutical compositions of the invention may include any further agent which is used in the prevention or treatment of pulmonary hypertension. For example, the anti-pulmonary hypertension may include supplemental oxygen, diuretics, anticoagulants, calcium-channel blockers such as intravenous epoprostenol, adenosine, nifedipine, diltiazem, amlodipine, or inhaled nitric oxide, prostanoids such as prostacyclin derivatives, epoprostenol, treprostinil, iloprost, treprostinil, beraprost, prostaglandins, prostacyclin (prostaglandin 12), endothelin receptor antagonists such as bosentan, Sitaxsentan, ambrisentan and Actelion-1, nitric oxide and phosphodiesterase-5 inhibitors such as sildenafil and tadalafil. Said anti-pulmonary hypertension may include beta blockers, angiotensin-converting enzyme (ACE) inhibitors, digoxin, adenosine, vasoactive substances, guanylate cyclase activators (sGC), cinaciguat, riociguat, 5'-adenosine monophosphate-activated protein kinase (AMPK) activators, rho-kinase inhibitors, serotonin antagonists, phosphodiesterase-1 inhibitors, vasoactive intestinal peptide (VIP) and cyclophosphamide (Raiesdana and Loscalzo, 2006; Fukumoto and Shimokawa, 2011; Fuso et al., 2011).

Pharmaceutical compositions of the invention may include any further agent which is used in the prevention or treatment of lung inflammation, the inhibition of lung inflammation cell infiltrates or inhibition of cytokine expression.

In one embodiment, said additional active agents may be contained in the same composition or administrated separately.

In another embodiment, the pharmaceutical composition of the invention relates to combined preparation for simultaneous, separate or sequential use in the treatment of pulmonary hypertension.

Pharmaceutical compositions of the invention may include any further agent which has the capacity of limiting the cyclic nucleotides (cGMP, cAMP) elimination. Such agents may include but are not limited to specific phosphodiesterase (PDE) superfamily inhibitors including PDE3, PDE4 and PDE5 inhibitors. Examples of PDE4 inhibitors include rolipram and those described in patent documents US2005234238 DE10156229, DE10135009 and WO0146151. Examples of PDE5 inhibitors include sildenafil, vardenafil and tadalafil. Particularly preferred are PDE5 inhibitors that are marketed, e.g. VIAGRA(R) which is sildenafil citrate and which can be administered in this form. Other examples of PDE5 inhibitors also include those described in patent documents WO2005012303 and US2006106039.

Pharmaceutical compositions of the inventions may include any other antiproliferative agent that reduces smooth muscle cell proliferation. For example, the antiproliferative agent may be rapamycin, rapamycin derivatives, paclitaxel, docetaxel, 40-0-(3-hydroxy)propyl-rapamycin, 40-O-[2-(2-hydroxy)ethoxy]ethyl-rapamycin, and 40-O-tetrazole-rapamycin, ABT-578, everolimus and combinations thereof.

Pharmaceutical compositions of the invention may include compounds selected from the group consisting of antibodies, receptor ligands, enzymes, adhesion peptides, oligosaccharides, oligonucleotides and the like. Such compounds may be blood clotting factors, inhibitors or clot dissolving agents such as streptokinase and tissue plasminogen activator. Such agents can also include a prohealing drug that imparts a benign neointimal response characterized by controlled proliferation of smooth muscle cells and controlled deposition of extracellular matrix with complete luminal coverage by phenotypically functional (similar to uninjured, healthy intima) and morphologically normal (similar to uninjured, healthy intima) endothelial cells. Such compounds can also fall under the genus of antineoplastic, cytostatic, anti-inflammatory, antiplatelet, anticoagulant, antifibrin, antithrombin, antimitotic, antibiotic, antiallergic and antioxidant substances. Examples of such antineoplastics and/or antimitotics include paclitaxel (e.g. TAXOL(R) by Bristol-Myers Squibb Co., Stamford, Conn.), docetaxel (e.g. Taxotere(R), from Aventis S. A., Frankfurt, Germany) methotrexate, azathioprine, vincristine, vinblastine, fluorouracil, doxorubicin hydrochloride (e.g. Adriamycin(R) from Pharmacia & Upjohn, Peapack N. J.), and mitomycin (e.g. Mutamycin(R) from Bristol-Myers Squibb Co., Stamford, Conn.). Examples of such antiplatelets, anticoagulants, antifibrin, and antitlirombins include heparinoids, hirudin, recombinant hirudin, argatroban, forskolin, vapiprost, prostacyclin and prostacyclin analogues, dextran, D-phe-pro-arg-chloromethylketone (synthetic antithrombin), dipyridamole, glycoprotein Ilb/Illa platelet membrane receptor antagonist, antibody, and thrombin inhibitors such as Angiomax(R) (Biogen, Inc., Cambridge, Mass.). Examples of cytostatic agents include angiopeptin, angiotensin converting enzyme inhibitors such as captopril (e.g. Capoten and Capozide(R) from Bristol-Myers Squibb Co., Stamford, Conn.), cilazapril or lisinopril (e.g. Prinivil(R) and Prinzide(R) from Merck & Co., Inc., Whitehouse Station, N.J.), actinomycin D, or derivatives and analogs thereof. Synonyms of actinomycin D include dactinomycin, actinomycin IV, actinomycin II, actinomycin X1, and actinomycin Ci. Other compounds include calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor(R) from Merck & Co., Inc., Whitehouse Station, N.J.), monoclonal antibodies (such as those specific for Platelet-Derived Growth Factor (PDGF) receptors), nitroprusside, prostaglandin inhibitors, suramin, serotonin blockers, steroids, thioprotease inhibitors, triazolopyrimidine (a PDGF antagonist), and nitric oxide. An example of an antiallergic agent is permirolast potassium. Other therapeutic substances or agents which may be appropriate include alpha-interferon, genetically engineered epithelial cells, antibodies such as CD-34 antibody, abciximab (REOPRO), and progenitor cell capturing antibody, prohealing drugs that promotes controlled proliferation of muscle cells with a normal and physiologically benign composition and synthesis products, enzymes, anti-inflammatory agents, antivirals, anticancer drugs, anticoagulant agents, free radical scavengers, steroidal anti-inflammatory agents, non-steroidal anti-inflammatory agents, antibiotics, nitric oxide donors, super oxide dismutases, super oxide dismutases mimics, 4-amino- 2,2,6,6-tetramethylpiperidine-1-oxyl (4-amino-TEMPO), dexamethasone, clobetasol, aspirin, prodrugs thereof, co-drugs thereof, and a combination thereof. The foregoing substances are listed by way of example and are not meant to be limiting. Other active agents which are currently available or that may be developed in the future are equally applicable.

The present invention also relates to a kit for treating a pulmonary hypertension comprising a first pharmaceutical composition comprising a CCR5 antagonist and/or an inhibitor of CCR5 and a second pharmaceutical composition comprising one or more Phosphodiesterase (PDE) inhibitors selected from the group consisting of PDE3 inhibitors, PDE4 inhibitors, PDE5 inhibitors and mixtures thereof

Another object of the invention relates to a pharmaceutical composition according to the invention comprising one or more chemotherapeutic or radiotherapeutic agents.

In one embodiment said chemotherapeutic or radiotherapeutic agents are a therapeutic active agent used as anticancer agent. For example, said anticancer agents include but are not limited to fludarabine, gemcitabine, capecitabine, methotrexate, taxol, taxotere, mercaptopurine, thioguanine, hydroxyurea, cytarabine, cyclophosphamide, ifosfamide, nitrosoureas, platinum complexes such as cisplatin, carboplatin and oxaliplatin, mitomycin, dacarbazine, procarbizine, etoposide, teniposide, campathecins, bleomycin, doxorubicin, idarubicin, daunorubicin, dactinomycin, plicamycin, mitoxantrone, L-asparaginase, doxorubicin, epimbicm, 5-fluorouracil, taxanes such as docetaxel and paclitaxel, leucovorin, levamisole, irinotecan, estramustine, etoposide, nitrogen mustards, BCNU, nitrosoureas such as carmustme and lomustine, vinca alkaloids such as vinblastine, vincristine and vinorelbine, imatimb mesylate, hexamethyhnelamine, topotecan, kinase inhibitors, phosphatase inhibitors, ATPase inhibitors, tyrphostins, protease inhibitors, inhibitors herbimycm A, genistein, erbstatin, and lavendustin A. In one embodiment, additional anticancer agents may be selected from, but are not limited to, one or a combination of the following class of agents: alkylating agents, plant alkaloids, DNA topoisomerase inhibitors, anti-folates, pyrimidine analogs, purine analogs, DNA antimetabolites, taxanes, podophyllotoxin, hormonal therapies, retinoids, photo sensitizers or photodynamic therapies, angiogenesis inhibitors, antimitotic agents, isoprenylation inhibitors, cell cycle inhibitors, actinomycins, bleomycins, anthracyclines, MDR inhibitors and Ca²⁺ ATPase inhibitors.

Additional anticancer agents may be selected from, but are not limited to, cytokines, chemokines, growth factors, growth inhibitory factors, hormones, soluble receptors, decoy receptors, monoclonal or polyclonal antibodies, mono-specific, bi-specific or multi-specific antibodies, monobodies, polybodies.

Additional anticancer agent may be selected from, but are not limited to, growth or hematopoietic factors such as erythropoietin and thrombopoietin, and growth factor mimetics thereof.

Further therapeutic active agent can be an antiemetic agent. Suitable antiemetic agents include, but are not limited to, metoclopromide, domperidone, prochlorperazine, promethazine, chlorpromazine, trimethobenzamide, ondansetron, granisetron, hydroxyzine, acethylleucine monoemanolamine, alizapride, azasetron, benzquinamide, bietanautine, bromopride, buclizine, clebopride, cyclizine, dunenhydrinate, diphenidol, dolasetron, meclizme, methallatal, metopimazine, nabilone, oxypemdyl, pipamazine, scopolamine, sulpiride, tetrahydrocannabinols, thiefhylperazine, thioproperazine and tropisetron. In a preferred embodiment, the antiemetic agent is granisetron or ondansetron.

In another embodiment, the further therapeutic active agent can be an hematopoietic colony stimulating factor. Suitable hematopoietic colony stimulating factors include, but are not limited to, filgrastim, sargramostim, molgramostim and epoietin alpha.

In still another embodiment, the other therapeutic active agent can be an opioid or non-opioid analgesic agent. Suitable opioid analgesic agents include, but are not limited to, morphine, heroin, hydromorphone, hydrocodone, oxymorphone, oxycodone, metopon, apomorphine, nomioiphine, etoipbine, buprenorphine, mepeddine, lopermide, anileddine, ethoheptazine, piminidine, betaprodine, diphenoxylate, fentanil, sufentanil, alfentanil, remifentanil, levorphanol, dextromethorphan, phenazodne, pemazocine, cyclazocine, methadone, isomethadone and propoxyphene. Suitable non-opioid analgesic agents include, but are not limited to, aspirin, celecoxib, rofecoxib, diclofinac, diflusinal, etodolac, fenoprofen, flurbiprofen, ibuprofen, ketoprofen, indomethacin, ketorolac, meclofenamate, mefanamic acid, nabumetone, naproxen, piroxicam and sulindac.

In yet another embodiment, the further therapeutic active agent can be an anxiolytic agent. Suitable anxiolytic agents include, but are not limited to, buspirone, and benzodiazepines such as diazepam, lorazepam, oxazapam, chlorazepate, clonazepam, chlordiazepoxide and alprazolam.

The term "radiotherapeutic agent" as used herein, is intended to refer to any radiotherapeutic agent known to one of skill in the art to be effective to treat or ameliorate cancer, without limitation. For instance, the radiotherapeutic agent can be an agent such as those administered in brachytherapy or radionuclide therapy. Such methods can optionally further comprise the administration of one or more additional cancer therapies, such as, but not limited to, chemotherapies, and/or another radiotherapy.

### Screening method

In a further aspect, the present invention relates to a method of screening a candidate compound for use as a drug for the treatment of pulmonary hypertension in a subject in need thereof, wherein the method comprises the steps of: i) providing candidate compounds and ii) selecting candidate compounds that blocks CCR5 or inhibit CCR5 expression.

In a further aspect, the present invention relates to a method of screening a candidate compound for use as a drug for the treatment of pulmonary hypertension in a subject in need thereof, wherein the method comprises the steps of:
- providing a CCR5, providing the CCR5 ligand CCL5, providing a cell, tissue sample or organism expressing CCR5, providing pulmonary artery smooth muscle cells,
- providing a candidate compound such as small organic molecule, modified chemokines, antibodies, peptide or polypeptide,
- measuring the activity of CCR5,
- and selecting positively candidate compounds that blocks CCR5 or inhibit CCR5 expression.

Methods for measuring the activity of CCR5 are well known in the art. For example, measuring the CCR5 activity involves determining a Ki on the CCR5 cloned and transfected in a stable manner into a CHO cell line or measuring one or more of the second messengers of CCR5 (PI3K, AKT), or measuring CCL5-induced calcium flux, or measuring cell proliferation induced by the CCR5 ligand CCL5 in the presence or absence of the candidate compound.

Tests and assays for screening and determining whether a candidate compound is a CCR5 antagonist are well known in the art (Ness et al., 2006, U.S. Pat. No. 7,65,905, U.S. Appl. No. 09/834,996). In vitro and in vivo assays may be used to assess the potency and selectivity of the candidate compounds to reduce CCR5 activity. Briefly, said candidate compound may be assayed for their ability to compete with the natural ligands of CCR5 for receptor binding. For example a high throughput screen utilizing a CCR5 membrane binding assay identifies inhibitors of CCL5 (RANTES) binding. This assay utilizes membranes prepared from NIH 3T3 cells expressing the human CCR5 chemokine receptor which have the ability to bind to RANTES, a natural ligand for the receptor. Using a 96-well plate format, membrane preparations are incubated with ¹²⁵I-RANTES in the presence or absence of candidate compound for one hour. Candidate compounds are serially diluted over a wide range of 0.001 ug/ml to 1 ug/ml and tested in triplicates. Reaction cocktails are harvested through glass fiber filters, and washed thoroughly. Total counts for replicates are averaged and data reported as the concentration required to inhibit 50 percent of total ¹²⁵I-RANTES binding. Subsequent analyses may involve functional assays such as inhibition of calcium flux. Cells expressing the HIV coreceptor CCR5 are loaded with calcium sensitive dyes prior to addition of candidate compound or the natural CCR5 ligand. Compounds with agonist properties will induce a calcium flux signal in the cell, while CCR5 antagonists are identified as compounds which do not induce signaling by themselves but are capable of blocking signaling by the natural ligand RANTES. The chemotaxis assay is a functional assay which characterizes the agonist vs. antagonist properties of the candidate compounds. The assay measures the ability of a non-adherent murine cell line expressing human CCR5 (BaF-550) to migrate across a membrane in response to either candidate compounds or natural ligands (i.e., RANTES, MIP-1β). Cells migrate across the permeable membrane towards compounds with agonist activity. Compounds that are antagonists not only fail to induce chemotaxis, but are also capable of inhibiting cell migration in response to known CCR5 ligands.

Activities of the candidate compounds, their ability to bind CCR5 and their ability to inhibit CCR5 activity may be tested using isolated pulmonary artery smooth muscle cells expressing CCR5, CHO cell line cloned and transfected in a stable manner by the human CCR5.

Cells and pulmonary artery smooth muscle cells expressing another receptor than CCR5 may be used to assess selectivity of the candidate compounds.

### Prognostics and diagnostics methods according to the invention

A further aspect of the invention relates to a method of identifying a subject having or at risk of having or developing a pulmonary hypertension and/or determining whether the subject will respond to anti-pulmonary hypertension agents which comprises the step of analyzing a biological sample from said subject for:
(i) detecting the presence of a mutation in the gene encoding for CCR5 and/or its associated promoter, and/or
(ii) determining the level of expression of the gene encoding for CCR5 or the CCR5 protein level.

Typical techniques for detecting a mutation in the gene encoding for CCR5 may include restriction fragment length polymorphism, hybridisation techniques, DNA sequencing, exonuclease resistance, microsequencing, solid phase extension using ddNTPs, extension in solution using ddNTPs, oligonucleotide assays, methods for detecting single nucleotide polymorphism such as dynamic allele-specific hybridisation, ligation chain reaction, mini-sequencing, DNA "chips", allele-specific oligonucleotide hybridisation with single or dual-labelled probes merged with PCR or with molecular beacons, and others.

Analyzing the expression of the gene encoding for CCR5 may be assessed by any of a wide variety of well-known methods for detecting expression of a transcribed nucleic acid or translated protein.

In a preferred embodiment, the expression of the gene encoding for CCR5 is assessed by analyzing the expression of mRNA transcript or mRNA precursors, such as nascent RNA, of said gene. Said analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TaqMan), and probes arrays such as GeneChip(TM) DNA Arrays (AFFYMETRIX).

Advantageously, the analysis of the expression level of mRNA transcribed from the gene encoding for CCR5 involves the process of nucleic acid amplification, e. g., by RT-PCR (the experimental embodiment set forth in U. S. Patent No. 4,683, 202), ligase chain reaction (Barany, 1991), self sustained sequence replication (Guatelli et al., 1990), transcriptional amplification system (Kwoh et al., 1989), Q-Beta Replicase (Lizardi et al., 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another preferred embodiment, the expression of the gene encoding for CCR5 is assessed by analyzing the expression of the protein translated from said gene. Said analysis can be assessed using an antibody (e.g., a radio-labeled, chromophore- labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.) which binds specifically to the protein translated from the gene encoding for CCR5.

Said analysis can be assessed by a variety of techniques well known from one of skill in the art including, but not limited to, enzyme immunoassay (EIA), radioimmunoassay (RIA), Western blot analysis and enzyme linked immunoabsorbant assay (RIA).

The method of the invention may further comprise a step consisting of comparing the CCR5 expression level in the sample with a control, wherein detecting differential in the CCR5 expression level between the sample and the control is indicative of a risk of having or developing a pulmonary hypertension. The control may consist in sample associated with a healthy subject not afflicted with pulmonary hypertension or in a sample associated with a subject afflicted with pulmonary hypertension.

According to the invention, high CCR5 expression level is indicative of subject having or at risk of having or developing a pulmonary hypertension and low CCR5 expression level is indicative of subject not having or at risk of having or developing a pulmonary hypertension.

Accordingly, the present invention relates to a method for monitoring the efficacy of a treatment of subject afflicted with a pulmonary hypertension, said method comprising the steps consisting of:
i) diagnosis of pulmonary hypertension before said treatment by performing the method of the invention
ii) diagnosis of pulmonary hypertension after said treatment by performing the method of the invention
iii) and comparing the results determined at step i) with the results determined at step ii) wherein a difference between said results is indicative of the effectiveness of the treatment.

Methods of the invention can be applied for monitoring the treatment (e.g., drug compounds) of the subject. For example, the effectiveness of an agent to affect the CCR5 expression level (as herein after described) according to the invention can be monitored during treatments of subjects receiving pulmonary hypertension treatments.

The "pulmonary hypertension treatment" relate to any type of pulmonary hypertension therapy undergone by the pulmonary hypertension subjects previously to collecting the pulmonary hypertension tissue samples, including anticoagulants, supplemental oxygen, diuretics, calcium-channel blockers, prostanoids, endothelin-receptor antagonists, nitric oxide, phosphodiesterase-5 inhibitors and surgery, e.g. atrial septostomy, pulmonary endarterectomy and lung transplantation.

### Kits

A further object of the invention relates to kits for performing the methods of the invention, wherein said kits comprise a compound which is a CCR5 antagonist and/or an inhibitor of CCR5 expression for use in the prevention or treatment of pulmonary hypertension in a subject in need thereof.

A further object of the invention relates to kits comprising a compound which is a CCR5 antagonist and/or an inhibitor of CCR5 expression for use in a method for inhibiting smooth muscle cell proliferation in a subject afflicted with pulmonary hypertension.

A further object of the invention relates to kits comprising a compound which is a CCR5 antagonist and/or an inhibitor of CCR5 expression for use in the prevention or treatment of vascular remodeling in a subject in need thereof.

A further object of the invention relates to kits comprising a compound which is a CCR5 antagonist and/or an inhibitor of CCR5 expression for use in the method according to the invention wherein said subject is afflicted with pulmonary arterial hypertension or HIV infection.

A further object of the invention relates to kits comprising a compound which is a CCR5 antagonist and/or an inhibitor of CCR5 expression for use in the method according to the invention wherein said CCR5 antagonist is an antibody or an antibody fragment.

A further object of the invention relates to kits comprising a compound which is a CCR5 antagonist and/or an inhibitor of CCR5 expression for use in the method according to the invention wherein said inhibitor of CCR5 expression is a siRNA, a ribozyme, or an antisense oligonucleotide.

A further object of the invention relates to kits comprising a pharmaceutical composition according to the invention and a pharmaceutically acceptable carrier for use in the prevention or treatment of pulmonary hypertension in a subject in need thereof.

In a particular embodiment, the kit of the invention relates to a kit for identifying whether a subject has or is at risk of having or developing a pulmonary hypertension, comprising means for analyzing a biological sample from said subject for:
(i) detecting the presence of a mutation in the gene encoding for CCR5 and/or its associated promoter, and/or
(ii) determining the level of expression of the gene encoding for CCR5 or the CCR5 protein level.

In a particular embodiment, the kit of the invention further comprise means for comparing the CCR5 expression level in the sample with a control, wherein detecting differential in the CCR5 expression level between the sample and the control is indicative of a risk of having or developing a pulmonary hypertension.

In another embodiment, the kit of the invention relates to a kit for monitoring the efficacy of a treatment of subject afflicted with a pulmonary hypertension, comprising means for:
i) diagnosis of pulmonary hypertension before said treatment by performing the method of the invention
ii) diagnosis of pulmonary hypertension after said treatment by performing the method of the invention
iii) and comparing the results determined at step i) with the results determined at step ii) wherein a difference between said results is indicative of the effectiveness of the treatment.

A further object of the invention relates to kits for performing the methods of the invention, wherein said kits comprise means for measuring the CCR5 expression level in the sample obtained from the subject. The kits may include probes, primers, macroarrays, microarrays and antibodies as above described.

For example, the kit may comprise a set of CCR5 mRNA probes as above defined, usually made of DNA, and that may be pre-labelled. Alternatively, probes may be unlabelled and the ingredients for labelling may be included in the kit in separate containers. The kit may further comprise hybridization reagents or other suitably packaged reagents and materials needed for the particular hybridization protocol, including solid-phase matrices, if applicable, and standards.

Alternatively the kit of the invention may comprise amplification primers (e.g. stem-loop primers) that may be pre-labelled or may contain an affinity purification or attachment moiety. The kit may further comprise amplification reagents and also other suitably packaged reagents and materials needed for the particular amplification protocol.

### Medical devices according to the invention

The present invention also relates to the use of a CCR5 antagonist and/or an inhibitor of CCR5 expression for the preparation of biomaterials or medical delivery devices selected among endovascular prostheses, such as stents, bypass grafts, internal patches around the vascular tube, external patches around the vascular tube, vascular cuff, and angioplasty catheter.

In this respect, the invention relates more particularly to biomaterials or medical delivery devices as mentioned above, coated with such CCR5 antagonist and/or an inhibitor of CCR5 expression as defined above, said biomaterials or medical devices being selected among endovascular prostheses, such as stents, bypass grafts, internal patches around the vascular tube, external patches around the vascular tube, vascular cuff, and angioplasty catheter. Such a local biomaterial or medical delivery device can be used to reduce stenosis as an adjunct to revascularizing, bypass or grafting procedures performed in any vascular location including coronary arteries, carotid arteries, renal arteries, peripheral arteries, cerebral arteries or any other arterial or venous location, to reduce anastomic stenosis such as in the case of arterial-venous dialysis access with or without polytetrafluoro- ethylene grafting and with or without stenting, or in conjunction with any other heart or transplantation procedures, or congenital vascular interventions.

For illustration purpose, such endovascular prostheses and methods for coating CCR5 antagonist and/or an inhibitor of CCR5 expression thereto are more particularly described in WO2005094916, or are those currently used in the art. The compounds used for the coating of the prostheses should preferentially permit a controlled release of said inhibitor. Said compounds could be polymers (such as sutures, polycarbonate, Hydron, and Elvax), biopolymers/biomatrices (such as alginate,fucans, collagen-based matrices, heparan sulfate) or synthetic compounds such as synthetic heparan sulfate-like molecules or combinations thereof. Other xamples of polymeric materials may include biocompatible degradable materials, e. g. lactone-based polyesters orcopolyesters, e. g. polylactide ; polylactide-glycolide ;polycaprolactone- glycolide ; polyorthoesters ; polyanhydrides ; polyaminoacids ; polysaccharides ;polyphospha- zenes; poly (ether-ester) copolymers, e. g. PEO-PLLA, or mixtures thereof; and biocompatible non-degrading materials, e. g. polydimethylsiloxane ; poly (ethylene-vinylacetate) ; acrylate based polymers or coplymers, e. g. polybutylmethacrylate, poly (hydroxyethyl methyl- methacrylate) ; polyvinyl pyrrolidinone ;fluorinated polymers such as polytetrafluoethylene ; cellulose esters. When a polymeric matrix is used, it may comprise 2 layers, e. g. a base layer in which said inhibitor is incorporated, such as ethylene-co-vinylacetate and polybutylmethacrylate, and a top coat, such as polybutylmethacrylate, which acts as a diffusion-control of said inhibitor. Alternatively, said inhibitor may be comprised in the base layer and the adjunct may be incorporated in the outlayer, or vice versa.

Such biomaterial or medical delivery device may be biodegradable or may be made of metal or alloy, e. g. Ni and Ti, or another stable substance when intented for permanent use. The CCR5 antagonist and/or an inhibitor of CCR5 expression of the invention may also be entrapped into the metal of the stent or graft body which has been modified to contain micropores or channels. Also internal patches around the vascular tube, external patches around the vascular tube, or vascular cuff made of polymer or other biocompatible materials as disclosed above that contain the inhibitor of the invention may also be used for local delivery.

Said biomaterial or medical delivery device allow the CCR5 antagonist and/or an inhibitor of CCR5 expression releasing from said biomaterial or medical delivery device over time and entering the surrounding tissue. Said releasing may occur during 1 month to 1 year. The local delivery according to the present invention allows for high concentration of the CCR5 antagonist and/or an inhibitor of CCR5 expression of the invention at the disease site with low concentration of circulating compound. The amount of said CCR5 antagonist and/or an inhibitor of CCR5 expression used for such local delivery applications will vary depending on the compounds used, the condition to be treated and the desired effect. For purposes of the invention, a therapeutically effective amount will be administered.

The local administration of said biomaterial or medical delivery device preferably takes place at or near the vascular lesions sites. The administration may be by one or more of the following routes: via catheter or other intravascular delivery system, intranasally, intrabronchially, interperitoneally or eosophagal. Stents are commonly used as a tubular structure left inside the lumen of a duct to relieve an obstruction. They may be inserted into the duct lumen in a non-expanded form and are then expanded autonomously (self-expanding stents) or with the aid of a second device in situ, e. g. a catheter-mounted angioplasty balloon which is inflated within the stenosed vessel or body passageway in order to shear and disrupt the obstructions associated with the wall components of the vessel and to obtain an enlarged lumen.

The biomaterial of the invention may be coated with any other compounds as above described for pharmaceutical compositions.

The invention will be further illustrated by the following figures and examples. However, these examples and figures should not be interpreted in any way as limiting the scope of the present invention.

### FIGURES:

**Figure 1****: CCR5 knock out mice are protected against development of pulmonary arterial hypertension induced by exposure to chronic hypoxia.**
   Right ventricular systolic pressure (RVSP) measurement in CCR5 +/+ and CCR5 -/mice maintained under normoxic conditions or exposed to hypoxia (10% 02).
**Figure 2****: CCR5 knock out mice are protected against development of pulmonary arterial hypertension induced by exposure to chronic hypoxia.**
   Fulton index measurement in CCR5 +/+ and CCR5 -/- mice maintained under normoxic conditions or exposed to hypoxia (10% 02).
**Figure 3****: Maraviroc protects against hypoxic pulmonary hypertension in CCR5 knock-in mice.**
   Treatment with Maraviroc protects against hypoxic pulmonary hypertension in CCR5 knock-in (KICCR5) mice but not in wild-type (WT) mice, as shown by a reduction of Right ventricular systolic pressure (RVSP) (A), Fulton index (B), % of muscularized vessels (C) and proliferative cells detected by ki67 immunostaining (D) following treatment with Maraviroc (200 mg/kg/day) in KICCR5 mice but not in WT mice. **p<0.01, ***p<0.001.

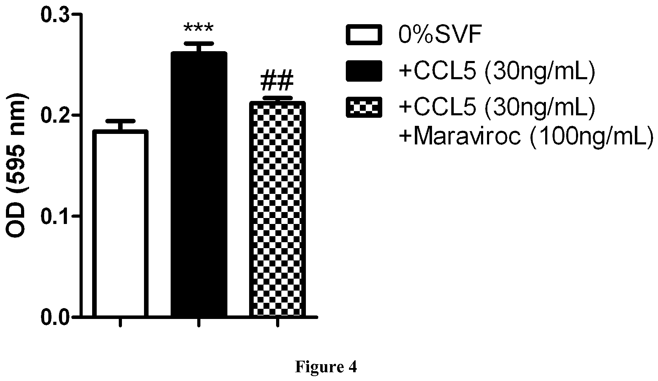

**Figure 4****: Pharmacological inhibition of CCR5 reduces proliferation of humain pulmonary artery smooth muscle cells.**
   Proliferation of human pulmonary artery smooth muscle cells stimulated by the CCR5 ligand CCL5 is significantly reduced (MTT assay) by pharmacological inhibition of CCR5 with Maraviroc.

### EXAMPLES:

### EXAMPLE 1:

### Materials and methods:

### Methods:

### Mice

Mice lacking CCR5 (CCR5-/-) were generated by homologous recombination on the C57B6/SV129 genetic background. The wild-type CCR5+/+ and mutant homozygous CCR5-/- mice used in these studies were siblings (8-10 weeks of age) obtained by breeding heterozygous mutants. Pups were typed by Southern blot analysis of tail biopsies. For the study of chronic hypoxia effects, mice were randomly divided into two groups, of which one was maintained in room air and the other exposed to hypoxia. All animal care and procedures were in accordance with institutional guidelines.

### Hemodynamic response of normoxic mice to acute hypoxia

Mice 6-10 weeks of age, weighing approximately 20-30 g were anesthetized with intraperitoneal ketamine (6 mg/100 g) and xylazine (1 mg/100 g). The trachea was cannulated, and the lungs were ventilated with room air at a tidal volume of 0.2 ml and a rate of 90 breaths per minute. A 26-gauge needle was then introduced percutaneously into the right ventricle via the subxyphoid approach. Right ventricular systolic pressure (RVSP) was measured using a Gould P10 EZ pressure transducer connected to pressure modules and a Gould TA 550 recorder. Right ventricular systolic pressure and heart rate were recorded first while the animal was ventilated with room air and then after 5 min of ventilation with the hypoxic gas mixture (8% O₂, 92% N₂). The heart rate under these conditions was between 300 and 500 bpm. If the heart rate fell below 300 bpm, measurements were excluded from analysis.

### Exposure to chronic hypoxia

Mice were exposed to chronic hypoxia (10% O₂) in a ventilated chamber (500-liter volume, Flufrance, Cachan, France). To establish the hypoxic environment, the chamber was flushed with a mixture of room air and nitrogen, and the gas was recirculated. The chamber environment was monitored using an oxygen analyzer (Servomex OA150, Crowborough, England). Carbon dioxide was removed by soda lime granules, and excess humidity was prevented by cooling of the recirculation circuit. The chamber temperature was maintained at 22 - 24°C. The chamber was opened every other day for 1 hour to clean the cages and replenish food and water supplies. Normoxic mice were kept in the same room, with the same light-dark cycle.

### Assessment of pulmonary hypertension

Mice previously exposed to hypoxia or room air for 2 or 5 weeks were anesthetized and ventilated with room air as described above. After incision of the abdomen and diaphragm, a 26-gauge needle connected to a pressure transducer was inserted into the right ventricle, and RVSP was recorded immediately. Next, blood was sampled for hematocrit determination and 5-HT measurements. Finally, after an intraperitoneal injection of sodium pentobarbital (40 mg/kg) and exsanguination, the thorax was opened and the lungs and heart were removed. The right ventricle (RV) was dissected from the left ventricle + septum (LV+S), and these dissected samples were weighed.

The lungs were fixed by intratracheal infusion of 4% aqueous buffered formalin at a pressure of 23 cm H₂O. The entire specimen was placed in a bath of the same fixative for one week. A midsagittal slice of the right lung including the apical, azygous and diaphragmatic lobes was processed for paraffin embedding. Sections (5 µm thick) were cut for light microscopy and stained with hematoxylin-phloxin-saffron and orcein-picroindigo-carmine.

In each mouse, a total of 50-60 intraacinar vessels accompanying either alveolar ducts or alveoli were analyzed by an observer blinded to the genotype and treatment. Each vessel was categorized as nonmuscular (no evidence of any vessel wall muscularization), partially muscular (SMCs identifiable in less than three-fourths of the vessel circumference), or fully muscular (SMCs in more than three-fourths of the vessel circumference). Muscularization was defined as the presence of typical SMCs stained red with phloxin, exhibiting an elongated shape and square-ended nucleus, and bound by two orcein-stained elastic laminae. The percentage of pulmonary vessels in each muscularization category was determined by dividing the number of vessels in that category by the total number counted in the same experimental group.

For fully muscular vessels, video images were obtained and arterial diameters were measured using computerized image analysis device. Percent wall thickness was then calculated as the diameter of the external elastic lamina minus the diameter of the internal lamina divided by the diameter of the external elastic lamina.

### Statistical analysis

All results are reported as means±SEMs. The nonparametric Mann-Whitney test was used to compare CCR5+/+ and CCR5-/- normoxic mice. Since in each genotype, hemodynamics, the right ventricular hypertrophy index, and muscularization did not differ across mice exposed to normoxia for various durations, the values of each of these parameters from normoxic mice were pooled. Two-way ANOVA was used to assess, in CCR5+/+ and CCR5-/- mice, the effects of hypoxia on hemodynamics, the right ventricular hypertrophy index, and muscularization.

### Results:

### Development of pulmonary hypertension and vascular remodeling

Under normoxic conditions, body weight (BW), RV weight /BW, hematocrit, and heart rate were similar in CCR5+/+ and CCR5-/- mice (n=6 in each group). Neither did mean systemic arterial pressure differ significantly between CCR5 +/+ and CCR5-/- mice.

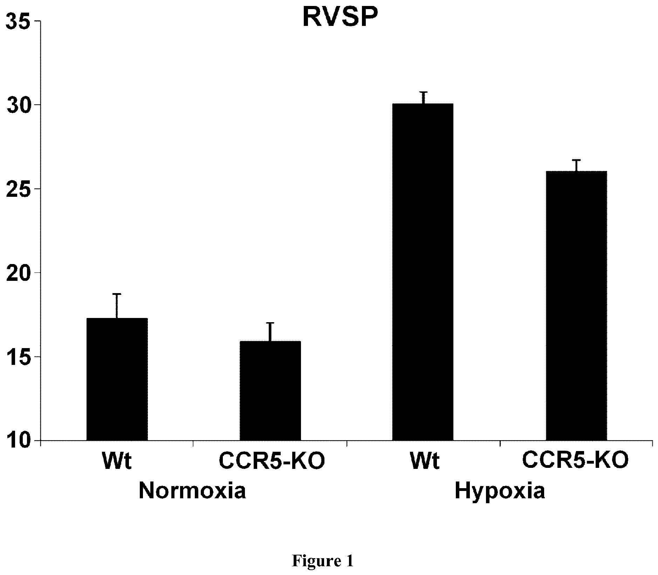

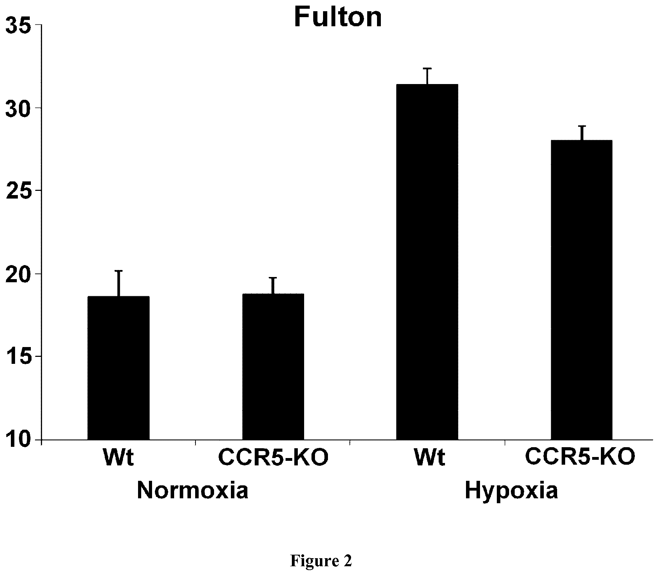

As compared with mice maintained under normoxic conditions, mice exposed to 10% O₂ exhibited gradual increases in RVSP, hematocrit, and Fulton Index. Whereas after exposure to hypoxia for similar durations, body weight, hematocrit, and heart rate did not differ between either genotype, RVSP was significantly lower and right ventricular hypertrophy less severe in CCR5-/- mutants than in CCR5+/+ mice (Figures 1 and 2). Furthermore, distal pulmonary vessel muscularization, which also increased with the duration of hypoxia exposure, was less marked at both the alveolar duct and the alveolar wall levels in CCR5-/- than in CCR5+/+ mice (P<0.01).

### EXAMPLE 2:

### Protocol and Methods:

To assess the inhibitory effects of the CCR5 antagonist Maraviroc on development of pulmonary hypertension in mice, the inventors used CCR5 knock-in mice in which the murine CCR5 gene was replaced by the human CCR5 gene. CCR5 knock-in mice were used in these experiments because Maraviroc binds to the human CCR5 but not to the murine CCR5 protein.

Male mice (12 months old), were exposed to chronic hypoxia (9% 02) in a ventilated chamber (Biospherix, New York, NY). Mice were anesthetized with ketamine (60 mg/Kg) and xylazine (10 mg/Kg) intraperitoneally. A 26-gauge needle connected to a pressure transducer was inserted into the right ventricle, and right ventricular systolic pressure (RVSP) was recorded. After the assessment of hemodynamic parameters, all mice were sacrificed by cardiac puncture and exsanguinations. The heart was dissected out and weighed for calculation of the Fulton index (ratio of right ventricular free wall weight over the sum of the septum plus left ventricular free wall weights: RV/LV+S). The left lung was inflated and fixed with formalin buffer for assessments of pulmonary vessel muscularization and of PA-SMC proliferation using Ki67 immunostaining.

### Results:

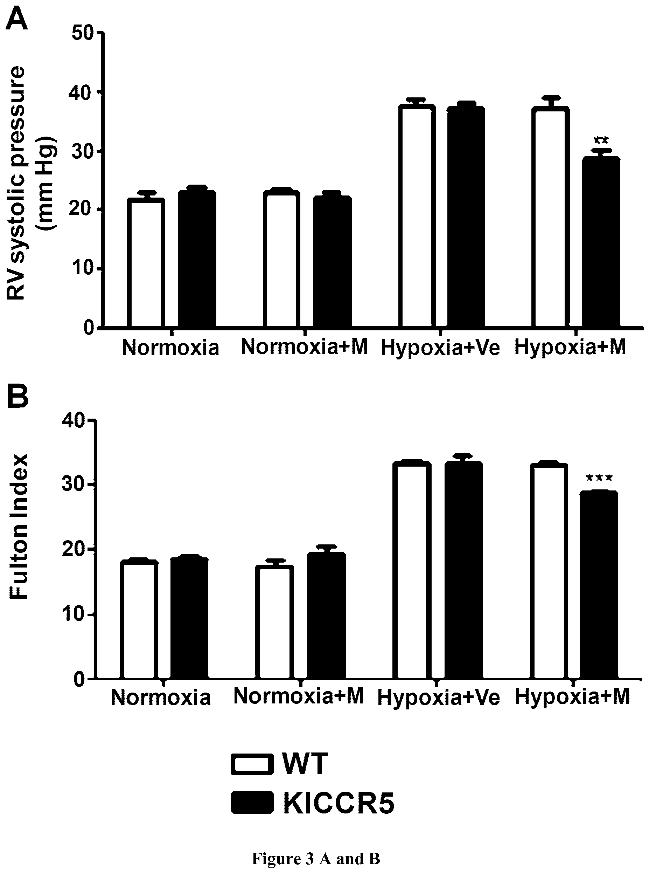

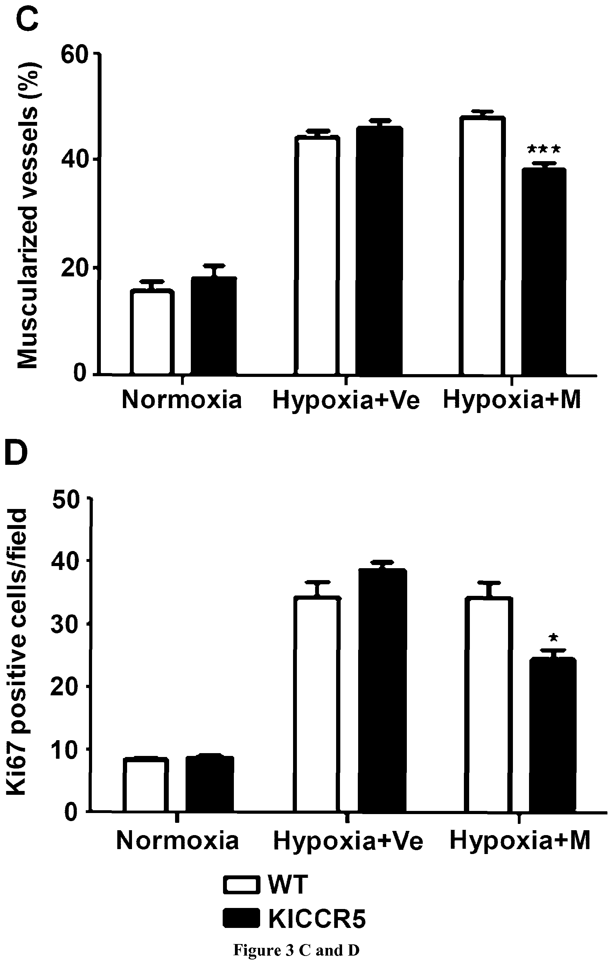

Treatment with Maraviroc protects against hypoxic pulmonary hypertension in CCR5 knock-in (KICCR5) mice but not in wild-type (WT) mice, as shown by a reduction of Right ventricular systolic pressure (RVSP) (Figure 3A), Fulton index (Figure 3B), % of muscularized vessels (Figure 3C) and proliferative cells detected by ki67 immunostaining (Figure 3D) following treatment with Maraviroc (200 mg/kg/day) in KICCR5 mice but not in WT mice (p<0.01, p<0.001).

### EXAMPLE 3:

### Protocol and Methods:

The effects of Maraviroc on cell proliferation was investigated in primary cultures of human pulmonary-artery smooth muscle cells (PA-SMCs) collected from lung tissue samples of patients undergoing lung resection surgery. Cell proliferation was assessed by using the tetrazolium salt (MTT) assay (Sigma, Lyon, France) during conditions of stimulation with the CCR5 ligand CCL5. Human PA-SMCs were seeded onto 24-well for 48h. PA-SMCs were synchronized for 48h in media without serum prior cell proliferation stimulation in presence of CCL5 or CCL5 and Maraviroc. 48 hours after, MTT (100 µg/mL) was added and incubated for 4 h. The media was removed and crystals formed were dissolved in 500 µL of dimethylsulfoxide (DMSO, Sigma) and the mixture was shaken for a few minutes to achieve complete dissolution. Aliquots (200 µL) of the resulting solutions were transferred to 96-well plates and absorbance was recorded at 520 nm using the Microplate Spectrophotometer System.

### Results:

Proliferation of human pulmonary artery smooth muscle cells stimulated by the CCR5 ligand CCL5 is significantly reduced (MTT assay) by pharmacological inhibition of CCR5 with Maraviroc (Figure 4).

### REFERENCES:

Throughout this application, various references describe the state of the art to which this invention pertains.
Barany F. Genetic disease detection and DNA amplification using cloned thermostable ligase. Proc Natl Acad Sci U S A. 1991; 88(1):189-93.
Biswas P, Tambussi G, Lazzarin A. Access denied? The status of co-receptor inhibition to counter HIV entry. Expert Opin Pharmacother. 2007; 8(7):923-33.
Brühl H, Cihak J, Stangassinger M, Schlöndorff D, Mack M. Depletion of CCR5-expressing cells with bispecific antibodies and chemokine toxins: a new strategy in the treatment of chronic inflammatory diseases and HIV. J Immunol. 2001; 166(4):2420-6.
Brummelkamp TR, Bernards R, Agami R. A system for stable expression of short interfering RNAs in mammalian cells. Science. 2002; 296(5567):550-3.
Colas P, Cohen B, Jessen T, Grishina I, McCoy J, Brent R. Genetic selection of peptide aptamers that recognize and inhibit cyclin-dependent kinase 2. Nature. 1996 ; 380(6574):548-50.
Elbashir SM, Lendeckel W, Tuschl T. RNA interference is mediated by 21- and 22-nucleotide RNAs. Genes Dev. 2001; 15(2):188-200.
Forbes IT, Cooper DG, Dodds EK, Hickey DM, Ife RJ, Meeson M, Stockley M, Berkhout TA, Gohil J, Groot PH, Moores K. CCR2B receptor antagonists: conversion of a weak HTS hit to a potent lead compound. Bioorg Med Chem Lett. 2000; 10(16):1803-6.
Fukumoto Y, Shimokawa H. Recent Progress in the Management of Pulmonary Hypertension. Circ J. 2011.
Fuso L, Baldi F, Di Perna A. Therapeutic strategies in pulmonary hypertension. Front Pharmacol. 2011; 2:21.
Guatelli JC, Whitfield KM, Kwoh DY, Barringer KJ, Richman DD, Gingeras TR. Isothermal, in vitro amplification of nucleic acids by a multienzyme reaction modeled after retroviral replication. Proc Natl Acad Sci U S A. 1990; 87(5):1874-8.
Hannon GJ. RNA interference. Nature. 2002; 418(6894):244-51.
Jayasena SD. Aptamers: an emerging class of molecules that rival antibodies in diagnostics. Clin Chem. 1999; 45(9):1628-50.
Köhler G, Milstein C. Continuous cultures of fused cells secreting antibody of predefined specificity. Nature. 1975; 256(5517):495-7.
Kwoh DY, Davis GR, Whitfield KM, Chappelle HL, DiMichele LJ, Gingeras TR. Transcription-based amplification system and detection of amplified human immunodeficiency virus type 1 with a bead-based sandwich hybridization format. Proc Natl Acad Sci U S A. 1989; 86(4):1173-7.
Latinovic O, Reitz M, Le NM, Foulke JS, Fätkenheuer G, Lehmann C, Redfield RR, Heredia A. CCR5 antibodies HGS004 and HGS101 preferentially inhibit drug-bound CCR5 infection and restore drug sensitivity of Maraviroc-resistant HIV-1 in primary cells. Virology. 2011; 411(1):32-40.
Lizardi PM, Guerra CE, Lomeli H, Tussie-Luna I, Kramer FR. Exponential amplification of recombinant-RNA hybridization probes. Biotechnology. 1988; 6: 1197-202.
Lopalco L. Natural anti-CCR5 antibodies in HIV-infection and -exposure. J Transl Med. 2011; 9 Suppl 1: S4.
McManus MT, Petersen CP, Haines BB, Chen J, Sharp PA. Gene silencing using micro-RNA designed hairpins. RNA. 2002; 8(6):842-50.
Ness TL, Kunkel SL, Hogaboam CM. CCR5 antagonists: the answer to inflammatory disease? Expert Opin Ther Pat. 2006; 16(8):1051-65.
Raiesdana A, Loscalzo J. Pulmonary arterial hypertension. Ann Med. 2006; 38(2):95-110.
Schanzer J, Jekle A, Nezu J, Lochner A, Croasdale R, Dioszegi M, Zhang J, Hoffmann E, Dormeyer W, Stracke J, Schäfer W, Ji C, Heilek G, Cammack N, Brandt M, Umana P, Brinkmann U. Development of tetravalent, bispecific CCR5 antibodies with antiviral activity against CCR5 monoclonal antibody-resistant HIV-1 strains. Antimicrob Agents Chemother. 2011; 55(5):2369-78.
Singh IP, Chauthe SK. Small molecule HIV entry inhibitors: Part I. Chemokine receptor antagonists: 2004 - 2010. Expert Opin Ther Pat. 2011; 21(2):227-69.
Simonneau G, Galiè N, Rubin LJ, Langleben D, Seeger W, Domenighetti G, Gibbs S, Lebrec D, Speich R, Beghetti M, Rich S, Fishman A. Clinical classification of pulmonary hypertension. J Am Coll Cardiol. 2004; 43(12 Suppl S):5S-12S.
Tuerk C, Gold L. Systematic evolution of ligands by exponential enrichment: RNA ligands to bacteriophage T4 DNA polymerase. Science. 1990; 249(4968):505-10.
Tuschl T, Zamore PD, Lehmann R, Bartel DP, Sharp PA. Targeted mRNA degradation by double-stranded RNA in vitro. Genes Dev. 1999; 13(24):3191-7.
Wu H, Nie Y, Huse WD, Watkins JD. Humanization of a murine monoclonal antibody by simultaneous optimization of framework and CDR residues. J Mol Biol. 1999; 294(1):151-62.
Yang H, Rotstein DM. Novel CCR5 antagonists for the treatment of HIV infection: a review of compounds patented in 2006 - 2008. Expert Opin Ther Pat. 2010; 20(3):325-54.

## Claims

1. A compound which is a CCR5 antagonist and/or an inhibitor of CCR5 expression for use in the prevention or treatment of pulmonary hypertension in a subject in need thereof.

2. A compound which is a CCR5 antagonist and/or an inhibitor of CCR5 expression for use in a method for inhibiting smooth muscle cell proliferation in a subject afflicted with pulmonary hypertension.

3. A compound which is a CCR5 inhibitor and/or an inhibitor of CCR5 expression for use in the prevention or treatment of vascular remodeling in a subject afflicted with pulmonary hypertension.

4. The compound according to any one of claims 1 to 3 wherein said subject is afflicted with pulmonary arterial hypertension.

5. The compound according to any one of claims 1 to 4 wherein said subject is afflicted with HIV infection.

6. The compound for use according to any one of claims 1 to 5 wherein said CCR5 antagonist is selected in the group consisting of maraviroc, NCB- 9471 , PRO-140, TAK-779, ZM-688523, 4-chloro-6-fluoro sulphonamide, UK-427857, TAK-220, TAK-652, SC-351125, ancriviroc, (SCH-C), SCH-351125 (SCH-C), SCH-417690 (SCH-D), vicroviroc, PRO-140, apliviroc (GW-873140, Ono-4128, AK-602), AMD-887, INC- B9471 , CMPD-167, methyl1-endo-{8-[(3S)-3-(acetylamino)-3-(3-fluorophenyl)propyl]-8-azabicyclo[3.2.1]oct-3-yl}-2-methyl-4,5,6,7-tetrahydro-1 H- imidazo[4,5-c]pyridine-5-carboxylate, methyl 3-endo-{8-[(3S)-3-(acetamido)-3-(3-fluorophenyl)propyl]-8- azabicyclo[3.2.1]oct-3-yl}-2-methyl-4,5,6,7-tetrahydro-3H-imidazo[4,5-c]pyridine-5-carboxylate, ethyl 1- endo-{8-[(3S)-3-(acetylamino)-3-(3-fluorophenyl)propyl]-8-azabicyclo[3.2.1]oct-3-yl}-2-methyl-4,5,6,7-tetrahydro-1H-imidazo[4,5-c]pyridine-5-carboxylate and N-{(1S)-3-[3-endo-(5-lsobutyryl-2-methyl-4,5,6,7-tetrahydro-1H-imidazo[4,5-c]pyridin-1-yl)-8-azabicyclo[3.2.1]oct-8-yl]-1-(3-fluorophenyl)propyl}acetamide) and pharmaceutically acceptable salts, solvates or derivatives of the above.

7. The compound for use according to any one of claims 1 to 5 wherein said CCR5 antagonist is an antibody or an antibody fragment.

8. The compound for use according to any one of claims 1 to 5 wherein said inhibitor of CCR5 expression is a siRNA, a ribozyme, or an antisense oligonucleotide.

9. A pharmaceutical composition comprising a compound according to any one of claims 1 to 8 and a pharmaceutically acceptable carrier for use in the prevention or treatment of pulmonary hypertension in a subject in need thereof.

10. A Kit comprising a compound according to any one of claims 1 to 8 or a pharmaceutical composition according to claim 9 for use in the prevention or treatment of pulmonary hypertension in a subject in need thereof.

11. A method of identifying a subject having or at risk of having or developing a pulmonary hypertension, which comprises the step of analyzing a biological sample from said subject for:
(i) detecting the presence of a mutation in the gene encoding for CCR5 and/or its associated promoter, and/or
(ii) determining the level of expression of the gene encoding for CCR5 or the CCR5 protein level.

12. The method according to claim 11 which further comprise a step consisting of comparing the CCR5 expression level in the sample with a control, wherein detecting differential in the CCR5 expression level between the sample and the control is indicative of a risk of having or developing a pulmonary hypertension.

13. The method according to any one of claims 11 or 12, for monitoring the efficacy of a treatment for a pulmonary hypertension.

14. A kit for identifying whether a subject has or is at risk of having or developing a pulmonary hypertension, which comprises means for analyzing a biological sample from said subject for:
(i) detecting the presence of a mutation in the gene encoding for CCR5 and/or its associated promoter, and/or
(ii) determining the level of expression of the gene encoding for CCR5 or the CCR5 protein level.

15. The kit according to claim 13 which further comprise means for comparing the level of the compound in the sample with a control, wherein detecting differential in the level of the compound between the sample and the control is indicative of a risk of having or developing a pulmonary hypertension.

## Patentansprüche

1. Verbindung, welche ein CCR5-Antagonist und/oder ein Inhibitor einer CCR5-Expresssion ist, zur Verwendung in der Vorbeugung oder Behandlung von pulmonaler Hypertonie in einem Subjekt, das dessen bedarf.

2. Verbindung, welche ein CCR5-Antagonist und/oder ein Inhibitor einer CCR5-Expresssion ist, zur Verwendung in einem Verfahren zum Inhibieren einer Proliferation glatter Muskelzellen in einem Subjekt, welches an pulmonaler Hypertonie leidet.

3. Verbindung, welche ein CCR5-Antagonist und/oder ein Inhibitor einer CCR5-Expresssion ist, zur Verwendung in der Vorbeugung oder Behandlung eines vaskulären Umbaus in einem Subjekt, welches an pulmonaler Hypertonie leidet.

4. Verbindung nach einem der Ansprüche 1 bis 3, wobei das Subjekt an pulmonaler arterieller Hypertonie leidet.

5. Verbindung nach einem der Ansprüche 1 bis 4, wobei das Subjekt an einer HIV-Infektion leidet.

6. Verbindung zur Verwendung nach einem der Ansprüche 1 bis 5, wobei der CCR5-Antagonist ausgewählt ist aus der Gruppe bestehend aus Maraviroc, NCB-9471 , PRO-140, TAK-779, ZM-688523, 4-Chlor-6-fluor-sulphonamid, UK-427857, TAK-220, TAK-652, SC-351125, Ancriviroc, (SCH-C), SCH-351125 (SCH-C), SCH-417690 (SCH-D), Vicroviroc, PRO-140, Apliviroc (GW-873140, Ono-4128, AK-602), AMD-887, INC-B9471 CMPD-167, Methyl-1-endo-{8-[(3S)-3-(acetylamino)-3-(3-fluorphenyl)propyl]-8-azabicyclo[3.2.1]oct-3-yl}-2-methyl-4,5,6,7-tetrahydro-1H-imidazo[4,5-c]pyridin-5-carboxylat, Methyl-3-endo-{8-[(3S)-3-(acetamido)-3-(3-fluorphenyl)propyl]-8-azabicyclo[3.2.1]oct-3-yl}-2-methyl-4,5,6,7-tetrahydro-3H-imidazo[4,5-c]pyridin-5-carboxylat, Ethyl-1-endo-{8-[(3S)-3-(acetylamino)-3-(3-fluorphenyl)propyl]-8-azabicyclo[3.2.1]oct-3-yl}-2-methyl-4,5,6,7-tetrahydro-1H-imidazo[4,5-c]pyridin-5-carboxylat und N-{(1S)-3-[3-endo-(5-isobutyryl-2-methyl-4,5,6,7-tetrahydro-1H-imidazo[4,5-c]pyridin-1-yl)-8-azabicyclo[3.2.1]oct-8-yl]-1-(3-fluorphenyl)propyl}acetamid) und pharmazeutisch verträglichen Salzen, Solvaten oder Derivaten der obigen.

7. Verbindung zur Verwendung nach einem der Ansprüche 1 bis 5, wobei der CCR5-Antagonist ein Antikörper oder ein Antikörperfragment ist.

8. Verbindung zur Verwendung nach einem der Ansprüche 1 bis 5, wobei der Inhibitor der CCR5-Expression eine siRNA, ein Ribozym, oder ein Antisense-Oligonukleotid ist.

9. Pharmazeutische Zusammensetzung umfassend eine Verbindung nach einem der Ansprüche 1 bis 8 und einen pharmazeutisch verträglichen Träger zur Verwendung in der Vorbeugung oder Behandlung von pulmonaler Hypertonie in einem Subjekt, das dessen bedarf.

10. Kit umfassend eine Verbindung nach einem der Ansprüche 1 bis 8 oder eine pharmazeutische Verbindung nach Anspruch 9 zur Verwendung in der Vorbeugung oder Behandlung von pulmonaler Hypertonie in einem Subjekt, das dessen bedarf.

11. Verfahren zum Identifizieren eines Subjekts, welches eine pulmonale Hypertonie aufweist oder ein Risiko aufweist, eine zu entwickeln, welches den Schritt den Analysieren einer biologischen Probe von dem Subjekt umfasst, zum:
(i) Nachweisen des Vorliegens einer Mutation in dem Gen, welches CCR5 kodiert und/oder dessen assoziierten Promoter, und/oder
(ii) Feststellen des Expressionsniveaus des Gens, welches CCR5 kodiert, oder des CCR5-Proteinniveaus.

12. Verfahren nach Anspruch 11, welches ferner einen Schritt bestehend aus dem Vergleichen des CCR5-Expressionsniveaus in der Probe mit einer Kontrolle umfasst, wobei ein Nachweisen eines Unterschieds im CCR5-Expressionsniveaus zwischen der Probe und der Kontrolle das Risiko anzeigt, eine pulmonale Hypertonie aufzuweisen oder zu entwickeln.

13. Verfahren nach einem der Ansprüche 11 oder 12, zum Überwachen der Wirksamkeit einer Behandlung von pulmonaler Hypertonie.

14. Kit zum Identifizieren, ob ein Subjekt eine pulmonaler Hyertonie aufweist oder ein Risiko aufweist, eine zu entwickeln, welches Mittel zum Analysieren einer biologischen Probe aus dem Subjekt umfasst, zum:
(i) Nachweisen des Vorliegens einer Mutation in dem Gen, welches CCR5 kodiert und/oder dessen assoziierten Promoter, und/oder
(ii) Feststellen des Expressionsniveaus des Gens, welches CCR5 kodiert, oder des CCR5-Proteinniveaus.

15. Kit nach Anspruch 13, welches ferner Mittel zum Vergleichen des Niveaus der Verbindung in der Probe mit einer Kontrolle umfasst, wobei das Nachweisen eines Unterschieds im CCR5-Expressionsniveaus zwischen der Probe und der Kontrolle das Risiko anzeigt, eine pulmonale Hypertonie aufzuweisen oder zu entwickeln.

## Revendications

1. Composé qui est un antagoniste de CCR5 et/ou un inhibiteur d'expression de CCR5 à utiliser dans la prévention ou le traitement de l'hypertension pulmonaire chez un sujet en ayant besoin.

2. Composé qui est un antagoniste de CCR5 et/ou un inhibiteur d'expression de CCR5 à utiliser dans une méthode pour inhiber la prolifération de cellule musculaire lisse chez un sujet atteint d'hypertension pulmonaire.

3. Composé qui est un inhibiteur de CCR5 et/ou un inhibiteur d'expression de CCR5 à utiliser dans la prévention ou le traitement du remodelage vasculaire chez un sujet atteint d'hypertension pulmonaire.

4. Composé selon l'une quelconque des revendications 1 à 3, dans laquelle ledit sujet est atteint d'hypertension artérielle pulmonaire.

5. Composé selon l'une quelconque des revendications 1 à 4, dans laquelle ledit sujet est atteint d'une infection par VIH.

6. Composé à utiliser selon l'une quelconque des revendications 1 à 5, dans laquelle ledit antagoniste de CCR5 est sélectionné dans le groupe constitué de maraviroc, NCB-9471, PRO-140, TAK-779, ZM-688523, 4-chloro-6-fluoro sulfamide, UK-427857, TAK-220, TAK-652, SC-351125, ancriviroc, (SCH-C), SCH-351125 (SCH-C), SCH-417690 (SCH-D), vicroviroc, PRO-140, apliviroc (GW-873140, Ono-4128, AK-602), AMD-887, INC-B9471, CMPD-167, methyl1-endo-{8-[(3S)-3-(acétylamino)-3-(3-fluorophényl)propyl]-8-azabicyclo[3.2.1]oct-3-yl}-2-methyl-4,5,6,7-tétrahydro-1H-imidazo[4,5-c]pyridine-5-carboxylate, méthyle 3-endo-{8-[(3S)-3-(acétamido)-3-(3-fluorophényl)propyl]-8-azabicyclo[3.2.1]oct-3-yl}-2-methyl-4,5,6,7-tétrahydro-3H-imidazo[4,5-c]pyridine-5-carboxylate, éthyle 1-endo-{8-[(3S)-3-(acétylamino)-3-(3-fluorophényl)propyl]-8-azabicyclo[3.2.1]oct-3-yl}-2-méthyl-4,5,6,7-tétrahydro-1H-imidazo[4,5-c]pyridine-5-carboxylate et N-{(1S)-3-[3-endo-(5-isobutyryl-2-méthyl-4,5,6,7-tétrahydro-1H-imidazo[4,5-c]pyridin-1-yl)-8-azabicyclo[3.2.1]oct-8-yl]-1-(3-fluorophényl)propyl}acétamide) et des sels, solvates ou dérivés des précédents acceptables d'un point de vue pharmaceutique.

7. Composé à utiliser selon l'une quelconque des revendications 1 à 5, dans laquelle ledit antagoniste de CCR5 est un anticorps ou un fragment d'anticorps.

8. Composé à utiliser selon l'une quelconque des revendications 1 à 5, dans laquelle ledit inhibiteur d'expression de CCR5 est un petit ARN interférent, un ribozyme, ou un oligonucléotide antisens.

9. Composition pharmaceutique comprenant un composé selon l'une quelconque des revendications 1 à 8 et un vecteur acceptable d'un point de vue pharmaceutique à utiliser dans la prévention ou le traitement de l'hypertension pulmonaire chez un sujet en ayant besoin.

10. Kit comprenant un composé selon l'une quelconque des revendications 1 à 8 ou une composition pharmaceutique selon la revendication 9 à utiliser dans la prévention ou le traitement de l'hypertension pulmonaire chez un sujet en ayant besoin.

11. Procédé d'identification d'un sujet ayant ou en danger d'avoir ou de développer une hypertension pulmonaire, qui comprend l'étape d'analyse d'un échantillon biologique provenant dudit sujet pour :
(i) détecter la présence d'une mutation dans le gène codant pour CCR5 et/ou son promoteur associé, et/ou
(ii) déterminer le niveau d'expression du gène codant pour CCR5 ou le niveau protéique de CCR5.

12. Procédé selon la revendication 11, qui comprend en outre une étape consistant à comparer le niveau d'expression de CCR5 dans l'échantillon à un témoin, dans lequel la détection d'un différentiel dans le niveau d'expression de CCR5 entre l'échantillon et le témoin est indicative d'un risque d'avoir ou de développer une hypertension pulmonaire.

13. Procédé selon l'une quelconque des revendications 11 ou 12, pour contrôler l'efficacité d'un traitement pour une hypertension pulmonaire.

14. Kit pour identifier si un sujet a ou est en danger d'avoir ou de développer une hypertension pulmonaire, qui comprend des moyens pour analyser un échantillon biologique provenant dudit sujet pour :
(i) détecter la présence d'une mutation dans le gène codant pour CCR5 et/ou son promoteur associé, et/ou
(ii) déterminer le niveau d'expression du gène codant pour CCR5 ou le niveau protéique de CCR5.

15. Kit selon la revendication 13 qui comprend en outre des moyens pour comparer le niveau du composé dans l'échantillon à un témoin, dans lequel la détection d'un différentiel dans le niveau du composé entre l'échantillon et le témoin est indicative d'un risque d'avoir ou de développer une hypertension pulmonaire.
